# EUROPEAN PATENT SPECIFICATION

(11) **EP 1 972 310 B1**
(45) Date of publication and mention of the grant of the patent: **18.05.2011**
(21) Application number: 07118146.5
(22) Date of filing: 09.10.2007
(51) Int. Cl.: A61F 5/41, A61F 6/04, A61H 19/00

(54) **Condom assembly having an effect of enlarging and hardening penis for enhancing sensuality during intercourse**
Kondomanordnung mit Penisvergrößerungs- und Versteifungseffekt für intensivere Sinneslust während des Geschlechtsverkehrs
Ensemble préservatif ayant un effet sur l'agrandissement et le durcissement du pénis et améliorant la sensualité pendant le rapport sexuel

(30) Priority: 03.05.2007 US 744114; 19.03.2007 CN 200710087491
(43) Date of publication of application: 24.09.2008
(73) Proprietor: Ou, Wen-Show, 802 Kaohsiung City (TW)
(72) Inventor: Ou, Wen-Show, 802 Kaohsiung City (TW)
(74) Representative: Lorente Berges, Ana

(56) References cited:
- WO-A-83/01574
- US-A1- 2004 077 925
- US-B1- 6 776 755

## Description

### RELATED APPLICATIONS

### FIELD OF THE INVENTION

The present invention relates to condoms and more particularly to a condom equipped with an inner adhesive band provided with granules to enable a penis wearing the condom to have an effect of increased diameter and hardness due to the granules, thereby stimulating and enhancing the sensual pleasure during sexual intercourse.

### BACKGROUND OF THE INVENTION

To enhance sensual pleasure during sexual intercourse, condoms having a slightly roughened surface are available to consumers. However, the effect of such condoms is very limited. Attempts have also been made to adhere granules to the outer wall of condoms, however, granules cannot be introduced to condoms during the manufacturing process. Besides, a more serious problem is that condoms adhered with outer granules cannot pass various tests (such as water leak test, air-burst test, electric conductance test, etc.) required by law in order for condoms to effectively prevent pregnancy or venereal infection.

Operations have also been performed to embed beads into penis to enhance sensuality during intercourse. However, such operations involve many risks, especially for people with diabetes. What's worse, if the operation fails, the penis may develop erectile dysfunction, festering sores or inflammation. Moreover, a penis after such operation has beads in fixed size, and therefore cannot fit vaginas of different sizes. As a result, some people had to undergo a reverse operation to remove the beads.

According to medical studies, the average penis size of Oriental males is smaller in diameter than that of Caucasian males, but Caucasians' penises are not as erectile as Orientals'. In general, the diameter of the penis of an Oriental male is about 3.5 cm, whereas the diameter of the vagina of an Oriental female is about 3 cm for a virgin and 4 cm for a nonvirgin, respectively. However, the vagina after childbirth becomes lax and its diameter may increase to 5-6 cm. As a result, couples may not enjoy sex as much as before.

Some medical surveys indicate that about one-third of all females never experienced an orgasm. Besides, many women have a disorder called inhibited sexual desire (i.e. sexual apathy), which adversely affects their sexual desire. Condoms having an effect of increasing the diameter and the hardness of a penis for enhancing sensuality during sexual intercourse, such as those disclosed in the present application, should help alleviate these problems.

An example of such a condom is disclosed in US 2004/0077925.

### BRIEF SUMMARY OF THE INVENTION

The primary object of the present invention is to provide a condom assembly which can achieve the effect of increasing the diameter and hardness of the penis wearing it in order to stimulate and enhance the sensitivity and sensuality during sexual intercourse. Incidentally, since the condom of the present invention could contribute to higher sexual satisfaction between married couples, especially after childbirth, it might help reduce the incidence of extra-marital affairs.

To achieve the above object, the condom assembly of the present application comprises a flexible tubular sheath and a length of adhesive tape. The adhesive tape is fixedly provided with a plurality of granules on its outer non-adhesive surface. In one aspect, the condom of the present invention may be considered as a prior art condom provided with a length of adhesive tape having granules fixed on its outer surface. In use, the adhesive tape is wrapped around and adhered to an erectile penis to form a band just below the glans of the penis with the granules facing outwards; then the flexible tubular sheath is worn over the penis and the granules. Because the granules are enclosed by the flexible tubular sheath, the hardness of the former can be modulated by the latter. Thus, the flexible tubular sheath and the granules work together like a condom integrally formed with granules. Because the adhesive tape with granules is to be used inside the flexible tubular sheath (which may be a prior art condom), the condom of the present invention should have no difficulty passing the various tests for condoms. During sexual intercourse, the granules are pressed between the shaft of the penis and the vagina, causing the female to feel a relatively enlarged and hardened penis and therefore a heightened sensuality. Similarly, the male also feels a relatively tighter vagina due to the granules surrounding the penis. A vagina which has become lax due to childbirth could feel tight again. Therefore, couples or partners would be able to enjoy more in sexual intercourse. Moreover, if the granules come off as a result of the vigorous rubbing, the granules will stay within the tubular sheath and not fall into the vagina. The condom with an adhesive tape having the granular surface is not known in prior art; the invention is not obvious because no such combination was ever suggested in the condom industry.

### BRIEF DESCRIPTION OF THE DRAWINGS

FIG. 1 shows an adhesive tape having granules on one side thereof according to the present invention enclosing a male user's penis.
FIG. 2 illustrates the adhesive tape with a granular surface of FIG. 1 in use with a flexible tubular sheath having two open ends.
FIG. 3 illustrates the adhesive tape with a granular surface of FIG. 1 in use with a flexible tubular sheath having one closed end and one open end.
FIG. 4 shows a cross-sectional view of a penis wearing the adhesive tape with a granular surface of FIG. 1 and a flexible tubular sheath over the adhesive tape.
FIG. 5 shows a cross-sectional view of a penis wearing the adhesive tape with a granular surface of FIG. 1 and a flexible tubular sheath over the adhesive tape, as when inserted into a vagina.

### DETAILED DESCRIPTION OF THE INVENTION

The object and advantages of the condom assembly according to the present invention will be more readily understood from the following detailed description when read in conjunction with the appended drawings.

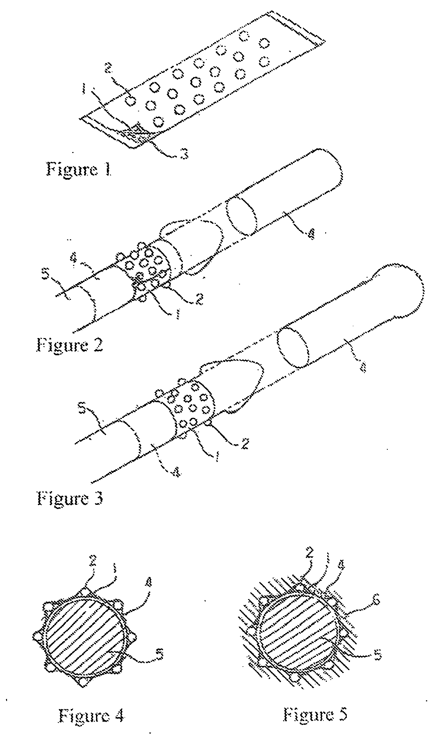

A preferred embodiment of the condom assembly according to the present invention is shown in Figs. 1 ~ 5. The condom assembly according to the present invention comprises a flexible tubular sheath 4 and an adhesive tape 1 with a plurality of granules 2 fixedly provided on the non-adhesive surface of the adhesive tape 1. The adhesive surface of the adhesive tape 1 is normally covered with a peelable protective layer 3. Before the adhesive tape 1 is used, the peelable protective layer 3 is peeled from the adhesive tape 1 to expose the adhesive surface of the adhesive tape 1.

The adhesive tape 1 excluding the granules 2 can be any tape made of paper, cloth, soft plastic or natural rubber molded into the form of a band or stripe, or any other shape. The granules 2 are made of a material with desired hardness; the size and shape of the granules 2 can be varied according to the desired effective diameter of a penis to be achieved. The granules 2 are not limited to any particular size, shape, hardness or material.

The flexible tubular sheath 4, like most conventional condoms, is made of an elastomeric membrane or natural latex. In one embodiment, as in Fig. 3, the flexible tubular sheath 4 has a closed end and an open end, just like a conventional condom.

In another embodiment, as in Fig. 2, the flexible tubular sheath 4 has two open ends. Although ineffective in preventing pregnancy or venereal infection, such design still works to enclose the granules 2 for enhancing sensuality and prevent the granules 2 from falling into the vagina during sexual intercourse.

In use, the adhesive tape 1 with the granules 2 is wrapped around and adhered to an erectile penis 5 slightly below the glans of the penis 5; the flexible tubular sheath 4 is then worn over the penis 5 and the granules 2. Because the granules 2 are tightly wrapped inside the flexible tubular sheath 4, the flexible tubular sheath 4 and the granules 2 work together like a condom integrally formed with granules. During sexual intercourse, the granules 2 are pressed between the penis 5 and the vagina, causing the female to feel a relatively enlarged and hardened penis 5 and a heightened sensuality due to the granules 2; at the same time, the male will also feel a relatively tighter vagina due to the granules 2 wrapped around the penis 5. A vagina which has become lax due to childbirth could feel tight again. Therefore, couples or partners would be able to enjoy more in sexual intercourse.

Moreover, in case the granules 2 come off as a result of the vigorous rubbing, the granules will 2 stay within the flexible tubular sheath 4 and not fall into the vagina.

In order that those skilled in the art can further understand the present invention, a detailed description of the present invention has been provided in the above. The present invention is thus described. It will be obvious that the same may be varied and modified in many ways. Such variations and modifications are not to be regarded as a departure from the scope of the present invention, and all such variations and modifications as would be obvious to one skilled in the art should be encompassed within the scope of the claims.

## Claims

1. A condom assembly having an effect of enlarging and hardening penis, said assembly comprising:
a flexible tubular sheath (4) having an open end and a closed end; and a length of adhesive tape (1) having a first adhesive surface and a second surface, **characterised in that** said second surface comprises a plurality of granules (2) fixedly provided thereon, whereby the length of adhesive tape is wrapped around and adhered to an erectile penis with the plurality of granules facing outwards before the flexible tubular sheath is worn over the penis and the plurality of granules such that granules, the hardness of which being modulated by the flexible tubular sheath, will stimulate and enhance sensuality during sexual intercourse.

2. The condom assembly as claimed in claim 1, wherein the flexible adhesive tape excluding the granules is made of paper, cloth, soft plastic, or natural rubber.

3. The condom assembly as claimed in claim 1, wherein the granules are made of a substance with a hardness within predetermined range.

4. The condom assembly as claimed in claim 1, wherein the first adhesive surface of the adhesive tape is covered with a peelable protective layer (3) which is to be peeled off before use.

5. A condom assembly having an effect of enlarging and hardening penis, said assembly comprising:
a flexible tubular sheath (4) having a first open end and a second open end; and a length of adhesive tape (1) having a first adhesive surface and a second surface **characterised in that** said second surface comprises a plurality of granules (2) fixedly provided thereon, whereby the length of adhesive tape is wrapped around and adhered to an erectile penis with the plurality of granules facing outwards before the flexible tubular sheath is worn over the penis and the plurality of granules such that the granules, the hardness of which being modulated by the flexible tubular sheath, will stimulate and enhance sensuality during sexual intercourse.

6. The condom assembly as claimed in claim 5, wherein the flexible adhesive tape excluding the granules is made of paper, cloth, soft plastic, or natural rubber.

7. The condom assembly as claimed in claim 5, wherein the granules are made of a substance with a hardness within a predetermined range.

8. The condom assembly as claimed in claim 5, wherein the first adhesive surface of the adhesive tape is covered with a peelable protective layer (3) which is to be peeled off before use.

## Patentansprüche

1. Ein Kondom, welches eine Vergrößerung und Verhärtung des Penis herbeiführt, bestehend aus:
einer flexiblen schlauchförmigen Hülle mit einem offenen Ende und einem geschlossenen Ende;
einem Streifen Klebeband mit einer ersten adhäsiven Oberfläche und einer zweiten Oberfläche, die mit einer Vielzahl von fest daran angebrachten Körnchenn versehen ist. Dabei wird der Streifen Klebeband so um einen erigierten Penis gewickelt wird, dass die Vielzahl der Körnchen nach außen weist, bevor die flexible schlauchförmigen Hülle über den Penis und die Vielzahl der Körnchen gezogen wird. Dadurch wird die Härte der besagten Körnchen durch die flexible schlauchförmige Hülle angemessen moduliert und es kommt zu einer Stimulation sowie zu einer verstärkten Gefühlsintensität während des Geschlechtsverkehrs.

2. Das Kondom, welches eine Vergrößerung und Verhärtung des Penis herbeiführt, wie in Anspruch 1, wobei das flexible Klebeband - ausgenommen der Körnchen - aus Papier, Stoff, weichem Kunststoff oder Naturkautschuk besteht.

3. Das Kondom, welches eine Vergrößerung und Verhärtung des Penis herbeiführt, wie in Anspruch 1, wobei die Körnchen aus einer Substanz hergestellt werden, deren Härtegrad in einem vorgegebenen Bereich liegt.

4. Das Kondom, welches eine Vergrößerung und Verhärtung des Penis herbeiführt, wie in Anspruch 1, wobei die erste Klebefläche des Klebebandes mit einer abziehbaren Schutzschicht abgedeckt ist, welche vor Gebrauch abzuziehen ist.

5. eine flexible schlauchförmige Hülle mit einem ersten offenen Ende und einem zweiten offenen Ende; einem Streifen Klebeband mit einer ersten adhäsiven Oberfläche und einer zweiten Oberfläche, die mit einer Vielzahl von fest daran angebrachten Körnchenn versehen ist. Dabei wird der Streifen Klebeband so um einen erigierten Penis gewickelt wird, dass die Vielzahl der Körnchen nach außen weist, bevor die flexible schlauchförmigen Hülle über den Penis und die Vielzahl der Körnchen gezogen wird. Dadurch wird die Härte der besagten Körnchen durch die flexible schlauchförmige Hülle angemessen moduliert und es kommt zu einer Stimulation sowie zu einer verstärkten Gefühlsintensität während des Geschlechtsverkehrs.

6. Das Kondom, welches eine Vergrößerung und Verhärtung des Penis herbeiführt, wie in Anspruch 1, wobei das flexible Klebeband - ausgenommen der Körnchen - aus Papier, Stoff, weichem Kunststoff oder Naturkautschuk besteht.

7. Das Kondom, welches eine Vergrößerung und Verhärtung des Penis herbeiführt, wie in Anspruch 1, wobei die Körnchen aus einer Substanz hergestellt werden, deren Härtegrad in einem vorgegebenen Bereich liegt.

8. Das Kondom, welches eine Vergrößerung und Verhärtung des Penis herbeiführt, wie in Anspruch 1, wobei die erste Klebefläche des Klebebandes mit einer abziehbaren Schutzschicht abgedeckt ist, welche vor Gebrauch abzuziehen ist.

## Revendications

1. Un préservatif avec effet de l'élargissement et le durcissement du pénis comprend:
une gaine tubulaire flexible ayant une extrémité ouverte et une extrémité opposée fermée; et un morceau de ruban adhésif ayant un côté collant et un côté granuleux. Avant de porter la gaine tubulaire flexible sur le pénis et le côté granuleux, enroulez le morceau de ruban adhésif autour et adhérez au pénis lors d'érection avec le côté granuleux vers l'extérieur. La dureté des granules sera modulée à stimuler et à renforcer la sensualité lors des rapports sexuels par la gaine tubulaire flexible.

2. Le préservatif avec effet de l'élargissement et le durcissement du pénis selon l'affirmation 1, dont le ruban adhésif flexible à l'exclusion des granules est fait de papier, en tissu, en plastique souple ou en caoutchouc naturel.

3. Le préservatif avec effet de l'élargissement et le durcissement du pénis selon l'affirmation 1, dont les granules sont faites d'une substance avec une dureté prédéterminée.

4. Le préservatif avec effet de l'élargissement et le durcissement du pénis selon l'affirmation 1, dont le côté collant du ruban adhésif est recouvert d'une couche de protection qui peut être décollé avant de l'utiliser.

5. Une gaine tubulaire flexible ayant une première extrémité ouverte et une seconde extrémité ouverte, et un morceau de ruban adhésif ayant un côté collant et un côté granuleux. Avant de porter la gaine tubulaire flexible sur le pénis et le côté granuleux, enroulez le morceau de ruban adhésif autour et adhérez au pénis lors d'érection avec le côté granuleux vers l'extérieur. La dureté des granules sera modulée à stimuler et à renforcer la sensualité lors des rapports sexuels par la gaine tubulaire flexible.

6. Le préservatif avec effet de l'élargissement et le durcissement du pénis selon l'affirmation 1, dont le ruban adhésif flexible à l'exclusion des granules est fait de papier, en tissu, en plastique souple ou en caoutchouc naturel.

7. Le préservatif avec effet de l'élargissement et le durcissement du pénis selon l'affirmation 1, dont les granules sont faites d'une substance avec une dureté prédéterminée.

8. Le préservatif avec effet de l'élargissement et le durcissement du pénis selon l'affirmation 1, dont le côté collant du ruban adhésif est recouvert d'une couche de protection qui peut être décollé avant de l'utiliser.
